# EUROPEAN PATENT SPECIFICATION

(11) **EP 2 566 388 B1**
(45) Date of publication and mention of the grant of the patent: **28.05.2014**
(21) Application number: 11717342.7
(22) Date of filing: 29.03.2011
(51) Int. Cl.: A61B 5/11, A61B 6/00, G01B 11/16, A61B 5/055

(54) **MOTION COMPENSATION AND PATIENT FEEDBACK IN MEDICAL IMAGING SYSTEMS**
BEWEGUNGSKOMPENSATION UND PATIENTENFEEDBACK IN EINEM MEDIZINISCHEN BILDGEBUNGSSYSTEM
COMPENSATION DE MOUVEMENT ET RETOUR D'INFORMATIONS AUPATIENT DANS DES SYSTEMES D'IMAGERIE MEDICALE

(30) Priority: 07.05.2010 US 332212 P
(43) Date of publication of application: 13.03.2013
(73) Proprietor: Koninklijke Philips N.V., 5656 AE Eindhoven (NL)
(72) Inventor: WANG, Jinnan, Briarcliff Manor, New York 10510-8001 (US); CHAN, Raymond, Briarcliff Manor, New York 10510-8001 (US); 'T HOOFT, Gert, Briarcliff Manor, New York 10510-8001 (US); DESJARDINS, Adrien Emmanuel, Briarcliff Manor, New York 10510-8001 (US); HALL, Christopher Stephen, Briarcliff Manor, New York 10510-8001 (US)
(74) Representative: van Velzen, Maaike Mathilde
(86) International application number: PCT/IB2011/051340
(87) International publication number: WO 2011/138691

(56) References cited:
- US-A1- 2006 013 523
- US-A1- 2010 215 311
- DE JONCKHEERE J ET AL: "OFSETH: Smart medical textile for continuous monitoring of respiratory motions under magnetic resonance imaging", 2009 ANNUAL INTERNATIONAL CONFERENCE OF THE IEEE ENGINEERING IN MEDICINE AND BIOLOGY SOCIETY : EMBC 2009 ; MINNEAPOLIS, MINNESOTA, USA, 3 - 6 SEPTEMBER 2009, IEEE, PISCATAWAY, NJ, USA, 3 September 2009 (2009-09-03), pages 1473-1476, XP031637942, ISBN: 978-1-4244-3296-7
- GRILLET A ET AL: "Optical Fiber Sensors Embedded Into Medical Textiles for Healthcare Monitoring", IEEE SENSORS JOURNAL, IEEE SERVICE CENTER, NEW YORK, NY, US, vol. 8, no. 7, 1 July 2008 (2008-07-01), pages 1215-1222, XP011231355, ISSN: 1530-437X, DOI: DOI:10.1109/JSEN.2008.926518
- A. GRILLET, D. KINET, J. WITT, M. SCHUKAR, K. KREBBER, F. PIROTTE, A. DEPR,: "Optical fibre sensors embedded into medical textiles for monitoring of respiratory movements in MRI environment", SPIE, PO BOX 10 BELLINGHAM WA 98227-0010 USA, vol. 6619, 66191R, 2007, pages 1-6, XP040242692, DOI: 10.1117/12.738631

## Description

The present invention generally relates to accurate and time-efficient motion tracking for time-resolved imaging and motion artifact compensation in imaging systems. The present invention specifically relates to an optical motion sensor incorporating one or more optical fibers embedded in a body contour conforming matrix.

Motion artifacts are caused by motion of an imaged object or a part of the imaged object during an imaging of the object. Thus, patient motion during a sequential acquisition of image frames of the patient may cause motion artifacts in the displayed image of the patient. More particularly, the patient may voluntary move or experience involuntary movements (e.g., respiration, cardiac motion and blood flow, eye movements and swallowing) during the image frame sequence, and such patient movements may result in blurring and/or ghosting within the displayed image of the patient. Accurate and time efficient motion tracking is therefore critical for motion compensation of motion artifacts in imaging systems.

However, motion tracking is highly dependent on the capabilities of the imaging system itself (e.g., an X-ray fluoroscopic system, a three-dimensional ultrasound system ("3D US"), a time-resolved magnetic resonance imaging system ("MRI"), and a computed tomography system ("CT")). Typically, motion tracking of this kind is limited by the imaging frame rate (e.g., 12Hz for 3D US) and can involve special acquisition sequences and processing overhead (e.g., tracking pulse sequence interleaved with imaging sequences in MRI). Additionally, for each imaging modality, there is further ability to integrate electrocardiogram, respiratory, and other physiological signals as gating triggers during image frame acquisition and/or image reconstruction. Motion tracking and subsequent compensation is therefore time consuming and is often less accurate than intrinsically high-frame rate imaging (e.g., motion compensated CT reconstruction vs. real-time echo for cardiac imaging).

The present invention, as compared to current motion tracking systems, offers an optical motion sensor for high accuracy, real time patient position information that can potentially increase image quality and shorten the image acquisition time. More particularly, the present invention allows for tracking and compensation of motions intrinsic to the target of interest that are directly reflected in the optical motion sensor.

One form of the present invention is an optical motion sensing system for use in imaging an anatomical structure. The optical motion sensing system employs the optical motion sensor and a motion tracker with the optical motion sensor including a body contour conforming matrix ("BCCM") and one or more optical fiber(s). For purposes of the present invention, the term "anatomical structure" is broadly defined herein as any structural feature of a human body or an animal body, whether external or internal or accessed externally or internally. Examples include, but are not limited to, body torso/limbs and organ systems.

Upon being adjoined to the anatomical structure, the BCCM structurally conforms, partially or entirely, to a surface contour of the anatomical structure for reciprocating any motion by the anatomical structure. More specifically, the BCCM may be affixed to or contiguous with the anatomical structure whereby the BCCM structural flexes, extends, expands, and/or compresses to conform, partially or entirely, to the surface contour of the anatomical structure. As such, the BCCM reciprocates, positively or negatively, any linear movement and/or angular movement by the anatomical structure, and/or any structural flexion, extension, expansion and/or compression by the anatomical structure.

Each optical fiber is embedded in the BCCM for generating an encoded optical signal indicative of a shape of the optical fiber(s) responsive to any reciprocal motion by the BCCM during an imaging of the anatomical structure.

The motion tracker is responsive to each encoded optical signal for periodically reconstructing the shape of the corresponding optical fiber(s). Each change in the shape of the optical fiber(s) between the geometric reconstructions of the optical fiber(s) represents motion by the anatomical structure during the imaging of the anatomical structure. For example, the motion tracker may reconstruct the shape of the optical fiber(s) for each frame or series of frames of an imaging of the anatomical structure whereby each change in the shape of the optical fiber(s) between each frame or series of frames represents linear and/or angular movement by the anatomical structure, and/or structural flexion, extension, expansion and/or compression by the anatomical structure.

The foregoing form and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defmed by the appended claims.

The following publication: J. De jonckheere et al. in "OFSETH: Smart medical textile for continuous monitoring of respiratory motions under magnetic resonance imaging", 2009 Annual International Conference of the IEEE Engineering in Medicine and Biology Society: EMBC 2009; 3-6 September 2009, Minneapolis, Minnesota, USA; IEEE, Piscataway, NJ, USA, 3rd September 2009, pages 1473-1476, ISBN: 978-1-4244-3296-7; discloses a motion sensing system according to the preamble of claim 1.
FIG. 1 illustrates an exemplary embodiment of a medical imaging system in accordance with present invention.
FIG. 2 illustrates an exemplary embodiment of an optical motion sensor in accordance with the present invention.
FIGS. 3 and 4 illustrate exemplary embodiments of an optical fiber as known in the art.
FIGS. 5 and 6 illustrate exemplary embodiments of the optical motion sensor shown in FIG. 2 in accordance with the present invention.
FIG. 7 illustrates an exemplary embodiment of the medical imaging system shown in FIG. 1 in accordance with the present invention.
FIG. 8 illustrates a flowchart representative of an exemplary embodiment of a motion compensation/patient feedback method in accordance with the present invention.

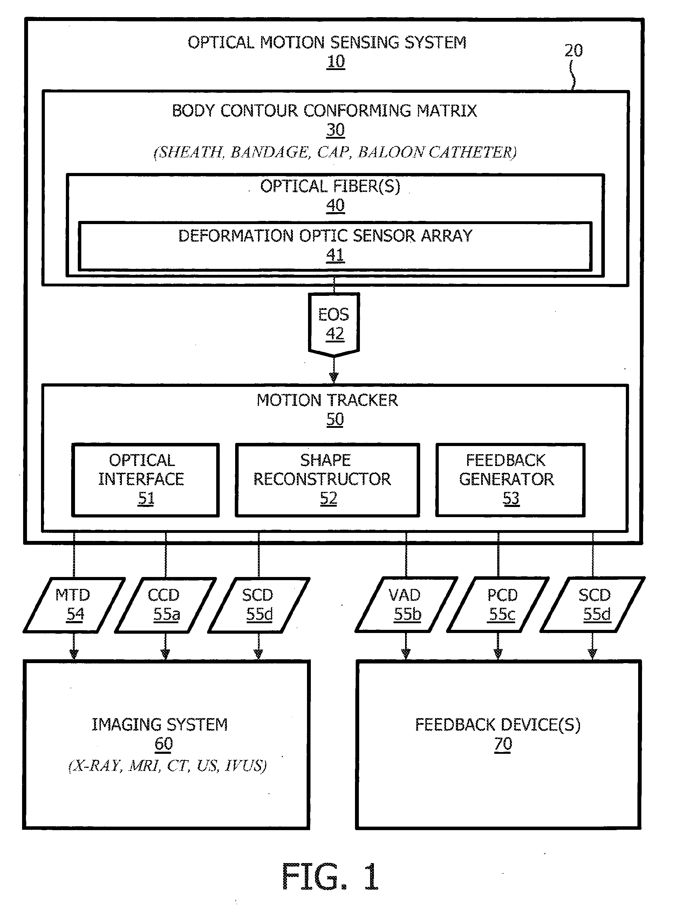

As shown in FIG. 1, a medical imaging system of the present invention employs an optical motion sensing system 10, an imaging system 60 (e.g., an X-ray device, MRI, CT, US, IVUS) and one or more feedback systems 70.

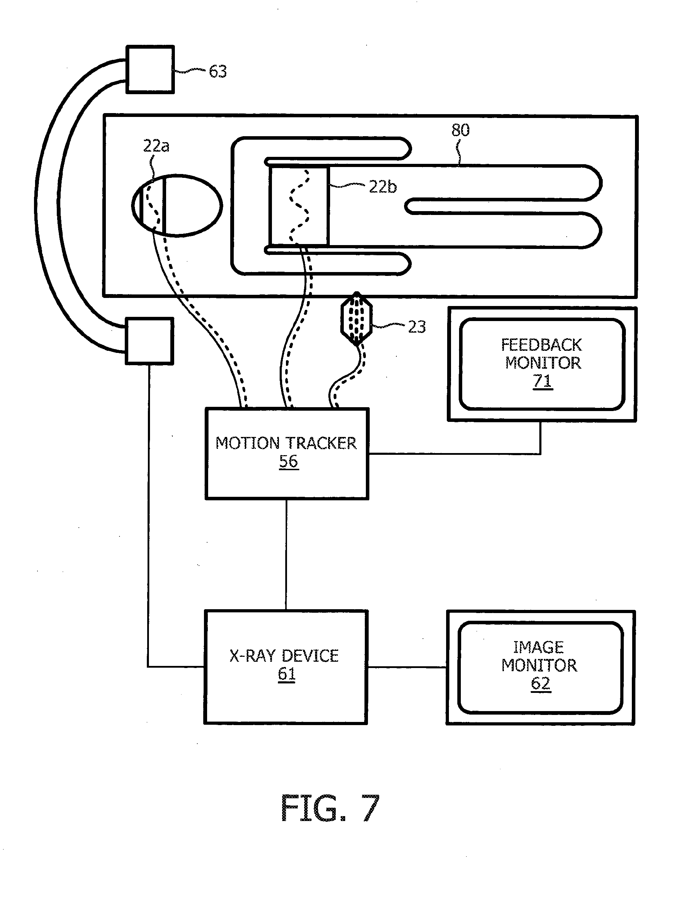

System 10 employs an optical motion sensor 20 including a BCCM 30, one or more optical fibers 40 and a motion tracker 50. For purposes of the present invention, BCCM 30 is broadly defined herein as any article or device structurally configured for structurally conforming, partially or entirely, to a surface contour of an anatomical structure upon being adjoined to the anatomical structure (e.g., affixed to or contiguous with the anatomical structure). By structurally conforming to the surface contour of the anatomical structure, any motion by the anatomical structure is reciprocated, positively or negatively, by BCCM 30 as will be further explained herein in connection with a description of FIG. 7. Examples of BCCM 30 include, but are not limited to, a sheath for encircling a human limb, a bandage for adhering to a human limb, a cap for covering a human head and a catheter balloon for abutting a human organ (e.g., a heart or an artery).

For purposes of the present invention, each optical fiber 40 is broadly defined herein as any article structurally configured for transmitting light by means of successive internal optical reflections via a deformation optic sensor array 41, and each deformation optic sensor array 41 is broadly defined herein as any article structurally configured for reflecting a particular wavelength of light while transmitting all other wavelengths of light whereby the reflection wavelength may be shifted as a function of an external stimulus applied to optical fiber 40. Examples of optical fiber 40 and deformation optic sensor array 41 include, but are not limited to, a flexible optically transparent glass or plastic fiber incorporating an array of fiber Bragg gratings integrated along a length of the fiber as known in the art, and a flexible optically transparent glass or plastic fiber having naturally variations in its optic refractive index occurring along a length of the fiber as known in the art.

In operation with one or more optical fibers 40 embedded within BCCM 30, each optical fiber 40 generates an encoded optical signal ("EOS") 42 via deformation optic sensor array 41 as known in the art that indicates a shape of optical fiber 40 at any instantaneous shape sampling of optical fiber 40 and more particularly over the course of multiple shape samplings, indicates each change to the shape of optical fiber 40 that occurs as BCCM 30 reciprocates motion of the anatomical structure. Encoded optical signal 42 therefore facilitates a use of optical fiber 40 in visually displaying a position and orientation of the anatomical structure within a defined space at any instantaneous time, and in visually displaying a motion tracking of the anatomical structure within the defined space.

To this end, system 10 further employs a motion tracker 50 incorporating an optical interface 51, a shape reconstructor 52 and a feedback generator 53 for processing encoded optical signal 42 to thereby periodically reconstruct a portion or an entire shape of optical fiber 40. For purposes of the present invention, optical interface 51 is broadly defined herein as any device or system structurally configured for transmitting light through optical fiber 40 to receive encoded optical signal 42 as generated by the successive internal reflections of the transmitted light via deformation optic sensor array 41. An example of optical interface 51 includes, but is not limited to, an arrangement of an optical coupler, a broadband reference reflector and a frequency domain reflectometer as known in the art for transmitting light through optical fiber 40 and for receiving encoded optical signal 42 as generated by the successive internal reflections of the transmitted light via deformation optic sensor array 41.

For purposes of the present invention, shape reconstructor 52 is broadly defined as any article or device structurally configured for processing encoded optic signal 42 to partially or entirely reconstruct the shape of optical fiber 40 and for generating motion tracking data ("MTD") 54a in an appropriate format for purposes of enabling imaging system 60 to compensate for motion-induced distortions in an imaging of the anatomical structure due to movement of the anatomical structure during the imaging of the anatomical structure. More particularly, any changes in the shape of an optical fiber 40 between geometrical reconstructions of the optical fiber 40 represent motion by the anatomical structure during the imaging by system 60. As such, motion compensation data 51a informs imaging system 60 of any sensed and tracked motion of the anatomical structure whereby imaging system 60 may execute known image-motion compensation algorithms to minimize, if not eliminate, any ghosting or blurring in the imaging of the anatomical structure. High accuracy image reconstruction by imaging system 60, particularly on a low-frame rate imaging, may therefore be achieved.

An example of shape reconstructor 52 includes, but is not limited to, a reconstruction engine installed as software and/or firmware on any type of computer for implementing a known shape reconstruction technique. In particular, a known shape reconstruction technique for correlating encoded optic signal 42 into strain/bend measurements that are integrated into a shape of optical fiber 40. In practice, the reconstruction engine may be integrated into imaging system 60.

For purposes of the present invention, feedback generator 53 is broadly defined herein as any article or device for converting motion tracking data 54 into feedback data 55 for purposes of providing motion feedback to imaging system 60 and one or more feedback systems 70.

In a first exemplary embodiment, feedback generator 53 may generate and communicate closed-loop control data ("CCD") 55a to imaging system 60 for purposes of enabling imaging system 60 to automatically adjust various imaging aspects of the system. For example, closed-loop control data 55a may facilitate automatic scan adjustments by imaging system 60 involving a determination of the position of the patient relative to imaging system 60 and reconstruction of high quality images of the anatomical structured based on the patient positioning.

In a second exemplary embodiment, feedback generator 53 may generate and communicate video/audio data ("VAD") 55b to a feedback system 70 having a monitor with speakers and/or earplugs for purposes of presenting the patient with visual and/or audio feedback as to any movement by the patient during the imaging of the anatomical structure. The patient therefore may adjust his/her body positioning, psychological state and/or physical state (e.g., breathing depth and rate) as needed to facilitate an accurate imaging of the anatomical structure. Furthermore, the visual/audio feedback may include information of maximum allowed motion for accurate imaging. For example, the visual feedback may include two (2) parallel lines and a dot with the lines indicating the maximum motion allowed and the dot indicates the current position of the patient, and the audio feedback may be presented whenever the patient motion is outside the parallel lines. The patent may therefore voluntarily adjust his/her position.

In a third exemplary embodiment, feedback generator 53 may generate and communicate patient control data ("PCD") 55c to a feedback system for counteracting movement by the patient and/or controlling a positioning of the patient. For example, patent control data 55c may be converted into forces to exert on the patient via BCCM 30 or any other article or device to counter any unacceptable movement by the patient. Also by example, patent control data 55c may be converted into control signals for an interventional device (e.g., a catheter) to maintain a positioning of the patient relative to imaging system 60.

In a fourth exemplary embodiment, feedback generator 53 may generate and communicate sensing control data ("SCD") 55d to a feedback system associated with imaging system 60 or another sensing system having a feedback system 70 (e.g., an electromagnetic tracking device for a surgical instrument). For example, sensing control data 55d may be converted into signals for executing position and/or orientation changes for certain sensing elements of the imaging system 60 (e.g., MR coils) or the sensing system to optimize image quality.

An example of feedback generator 53 includes, but is not limited to, a feedback engine installed as software and/or firmware on any type of computer for implementing one or more feedback modes. In practice, the feedback engine may be integrated into shape reconstructor 52, imaging system 60 or a feedback system 70.

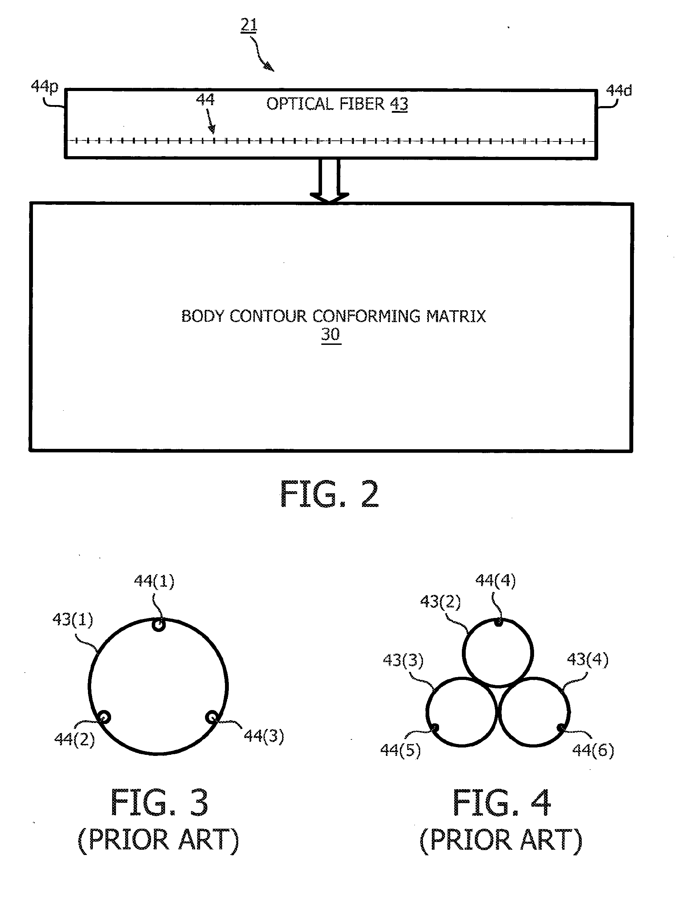

A description of FIGS. 2-8 will now be provided herein to facilitate a more detailed understanding of the present invention.

As shown in FIG. 2, an optical motion sensor 21 employs an optical fiber 43 embedded, partially or entirely, in BCCM 30. Optical fiber 43 includes one or more fiber Bragg grating arrays 44, each array 44 having a proximal end 44p and a distal end 44d. More particularly, for one version of optical fiber 43 shown in FIG. 3, optical fiber 43 has three (3) fiber Bragg grating arrays 44 arranged at 120° spacing as required for three-dimensional bend sensing. Alternatively, for another version of optical fiber 43 shown in FIG. 4, three (3) optical fibers 43 with each optical fiber 43 having a single fiber Bragg grating array 44 and the arrays 44 being arranged at 120° spacing as required for three-dimensional bend sensing.

The particular manner by which optical fiber 43 is embedded within BCCM 30 is dependent upon the application of optical motion sensor 21.

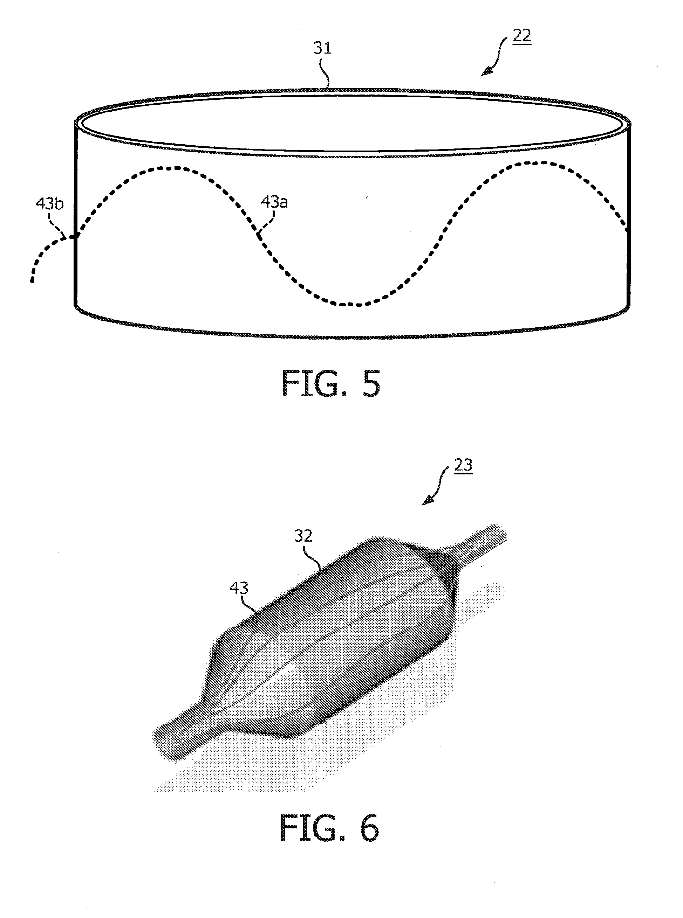

For example, as shown in FIG. 5, a portion 43a of an optical fiber 43 is embedded within a wall of a sheath version 31 of BCCM 30 to form an optical motion sensor 22 for encircling a limb or torso of a patient. More particularly, sheath 31 may encircle a head of a patient 80 as shown in FIG. 7. In this example, the shape of the portion 43a of optical fiber 43 within sheath 31 is fixed relative to the head of the patient once BCCM 30 is placed on the patient's head while the shape of the portion 43b of optical fiber 43 external to sheath 31 will change as the patient moves his/her head in any linear direction or any angular direction. This facilitates a motion tracking of various positions of the patient's head relative to the imaging device.

Alternatively or concurrently, sheath 31 may encircle a chest of patient 80 as shown in FIG. 7. The shape of the portion of optical fiber 43a within sheath 31 (FIG. 5) will change as the patient breathes while the shape of the portion of optical fiber 43a external to sheath 31 will change as the patient moves his/her chest in any linear direction or any angular direction. This facilitates a motion tracking of a depth and rate of breathing by the patient and of various positions of the patient's chest relative to the imaging device.

By further example, as shown in FIG. 6, several optical fibers 43 may extend in parallel through a wall of a catheter balloon version 32 of BCCM 30. The shape of a portion of optical fibers 43 extending through catheter balloon 32 will change as catheter balloon 32 is expanded or compressed as a negative reciprocation of an external stimulus being applied to catheter balloon 32 (e.g., a heart pressing against catheter balloon 32), while the shape of a portion of optical fibers 43 external to catheter balloon 32 (not shown) will change as catheter balloon 32 is moved in any linear direction or any angular direction. As such, the use of balloon 32 is appropriate for tracking rhythmic motion of organs (e.g., hearts and lungs).

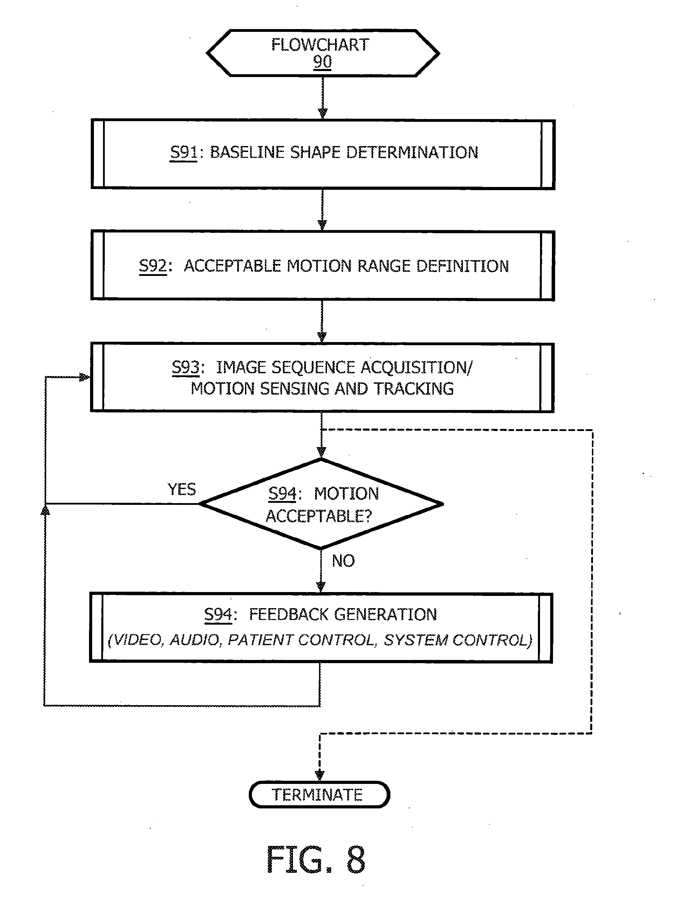

Referring to FIGS. 7 and 8, an exemplary operational use of the present invention as presented in an X-ray imaging of a patient 80 will now be described herein. First, a stage S91 of flowchart 90 encompasses a baseline shape determination of each optical fiber utilized in the procedure. For example, as shown in FIG. 7, an optical motion sensor 22a encircles a head of patient 80, an optical motion sensor 22b encircles a chest of patient 80 and a catheter balloon 23 is prepped to be inserted within patient 80. Prior to or at a time to of imaging patient 80 via an X-ray device 61, motion tracker 55 operates its optical interface and reconstruction engine to generate a baseline shape of the optical fibers of sensors 22 and 23.

A stage 92 of flowchart 90 encompasses a manual or an automatic definition of an acceptable range of motion of the anatomical structure(s) being tracked. Thus, for example, stage 92 may involve a definition of an acceptable range of linear movement and/or angular movements of the head and the chest of patient 80, an acceptable range of breathing depth and rate of patient 80, and an acceptable heart beat rate of patient 80.

A stage 93 of flowchart 90 encompasses an acquisition of an image sequence of the anatomical structure and a motion tracking for each frame or a series of frames (e.g., motion sensing/tracking every 100 frames) that facilitates a motion compensation for displaying an accurate imaging of patient 80 via an image monitor 62 and/or a closed-loop control of X-ray device 61 via a C-arm 63. Upon each reconstruction of a shape of the optical fibers, a stage 94 of flowchart 90 determines if any tracked motion of the subject anatomical structure is acceptable. If yes, then the next frame or series of frames is acquired. Otherwise, feedback as previously described herein is generated in the form of video, audio, patient control and/or system control during a stage 95 of flowchart 90.

For example, feedback monitor 71 may display two (2) parallel lines and a dot with the lines indicating the maximum motion allowed by a head of patient 80 and the dot indicates the current position of the head of patient 80. In addition, as represented by dashed lines extending from motion tracker 52 to sensors 22 and 23, feedback forces may be applied to patient 80 to control and/or maintain a positioning of patient 80.

While various exemplary embodiments of the present invention have been illustrated and described, it will be understood by those skilled in the art that the exemplary embodiments of the present invention as described herein are illustrative, and various changes and modifications may be made and equivalents may be substituted for elements thereof without departing from the true scope of the present invention. For example, although the invention is discussed herein with regard to FBGs, it is understood to include fiber optics for shape sensing or localization generally, including, for example, with or without the presence of FBGs or other optics, sensing or localization from detection of variation in one or more sections in a fiber using back scattering, optical fiber force sensing, fiber location sensors or Rayleigh scattering. In addition, many modifications may be made to adapt the teachings of the present invention without departing from its central scope. Therefore, it is intended that the present invention not be limited to the particular embodiments disclosed as the best mode contemplated for carrying out the present invention, but that the present invention includes all embodiments falling within the scope of the appended claims.

## Claims

1. An optical motion sensing system (10) for use in imaging an anatomical structure, the optical motion sensing system (10) comprising:
an optical motion sensor (20) including a body contour conforming matrix (30) and an optical fiber (40),
wherein, upon the body contour conforming matrix (30) being adjoined to the anatomical structure, the body contour conforming matrix (30) structurally conforms at least partially to a surface contour of the anatomical structure for reciprocating any motion by the anatomical structure, and
wherein the optical fiber (40) is at least partially embedded in the body contour conforming matrix for generating an encoded optical signal (42) indicative of a shape of the optical fiber (40) responsive to any reciprocal motion by the body contour conforming matrix (30) during an imaging of the anatomical structure; and **characterised by**
a motion tracker (50) responsive to the encoded optical signal (42) adapted to periodically reconstruct the geometrical shape of the optical fiber (40),
wherein each change in a shape of the optical fiber (40) between the geometric reconstructions of the optical fiber (40) represents motion by the anatomical structure during the imaging of the anatomical structure.

2. The optical motion sensing system (10) of claim 1, wherein an adjoining of the body contour conforming matrix (30) to the anatomical structure includes the body contour conforming matrix (30) being affixed to the anatomical structure.

3. The optical motion sensing system (10) of claim 1, wherein an adjoining of the body contour conforming matrix (30) to the anatomical structure includes the body contour conforming matrix (30) being contiguous with the anatomical structure.

4. The optical motion sensing system (10) of claim 1, wherein motion by the anatomical structure includes at least one of a linear movement of the anatomical structure, an angular movement by the anatomical structure, a structural flexion of the anatomical structure, a structural extension of the anatomical structure, a structural expansion of the anatomical structure and a structural compression of the anatomical structure.

5. The optical motion sensing system (10) of claim 1, wherein the body contour conforming matrix (30) is selected from a group including a sheath, a bandage, a cap and a catheter balloon.

6. The optical motion sensing system (10) of claim 1, wherein a portion of the optical fiber (40) embedded within the body contour conforming matrix (30) has a flexible shape responsive to any reciprocal motion by the body contour conforming matrix (30) during the imaging of the anatomical structure.

7. The optical motion sensing system (10) of claim 1,
wherein a first portion of the optical fiber (40) embedded within the body contour conforming matrix (30) has an inflexible shape unresponsive to any reciprocal motion by the body contour conforming matrix (30) during the imaging of the anatomical structure, and
wherein a second portion of the optical fiber (40) external to the body contour conforming matrix (30) has a flexible shape responsive to any reciprocal motion by the body contour conforming matrix (30) during the imaging of the anatomical structure.

8. A medical imaging system for imaging an anatomical structure, the medical imaging system comprising:
an optical motion sensing system as claimed in claim 1; and
an imaging system (60),
wherein the motion tracker (50) is operable to generate motion tracking data (54) representative of any motion by the anatomical structure during the imaging of the anatomical structure, and
wherein the imaging system (60) is responsive to the motion tracking data (54) for facilitating an execution of a motion compensation algorithm by the imaging system (60).

9. The medical imaging system of claim 8, wherein motion by the anatomical structure includes at least one of a linear movement of the anatomical structure, an angular movement by the anatomical structure, a structural flexion of the anatomical structure, a structural extension of the anatomical structure, a structural expansion of the anatomical structure and a structural compression of the anatomical structure.

10. The medical imaging system of claim 8, wherein the body contour conforming matrix (30) is selected from a group including a sheath, a bandage, a cap and a catheter balloon.

11. The medical imaging system of claim 8, wherein the motion tracker (50) is further operable to generate closed-loop control data (55a) for facilitating a closed-loop control of imaging system (60).

12. The medical imaging system of claim 8, further comprising:
a feedback system (70), wherein the motion tracker (50) is further operable to generate feedback data (55b) for providing at least visual feedback and audio feedback indicative of a motion of the anatomical structure.

13. The medical imaging system of claim 12, wherein the at least visual feedback and audio feedback indicates unacceptable motion by the anatomical structure.

14. The medical imaging system of claim 8, further comprising:
a feedback system (70), wherein the motion tracker (50) is further operable to generate patient control data (55c) for at least one of positioning the anatomical structure and maintaining a positioning of the anatomical structure.

15. The medical imaging system of claim 8, wherein the motion tracker (50) is further operable to generate system control data (55d) for adjusting at least one an operational aspect of the imaging system (60).

## Patentansprüche

1. Optisches Bewegungserfassungssystem (10), das zur Bildgebung einer anatomischen Struktur zum Einsatz kommt, wobei das optische Bewegungserfassungssystem (10) umfasst:
einen optischen Bewegungssensor (20) mit einer körperkonturkonformen Matrix (30) sowie einer optischen Faser (40),
wobei nach Anlegen der körperkonturkonformen Matrix (30) an die anatomische Struktur die körperkonturkonforme Matrix (30) zumindest zum Teil mit einer Oberflächenkontur der anatomischen Struktur übereinstimmt, um eine Bewegung seitens der anatomischen Struktur zu reziprokieren; und
wobei die optische Faser (40) zumindest teilweise in der körperkonturkonformen Matrix eingebettet ist, um ein codiertes optisches Signal (42) zu erzeugen, das auf eine Form der optischen Faser (40) schließen lässt, die auf eine reziproke Bewegung durch die körperkonturkonforme Matrix (30) während einer Bildgebung der anatomischen Struktur reagiert,
**gekennzeichnet durch**:
einen auf das codierte optische Signal (42) ansprechenden Motion-Tracker (Bewegungsnachführeinrichtung) (50), der so eingerichtet ist, dass er die geometrische Form der optischen Faser (40) periodisch rekonstruiert,
wobei jede Veränderung einer Form der optischen Faser (40) zwischen den geometrischen Rekonstruktionen der optischen Faser (40) eine Bewegung durch die anatomische Struktur während der Bildgebung der anatomischen Struktur darstellt.

2. Optisches Bewegungserfassungssystem (10) nach Anspruch 1, wobei ein Anlegen der körperkonturkonformen Matrix (30) an die anatomische Struktur das Befestigen der körperkonturkonformen Matrix (30) an der anatomischen Struktur beinhaltet.

3. Optisches Bewegungserfassungssystem (10) nach Anspruch 1, wobei ein Anlegen der körperkonturkonformen Matrix (30) an die anatomische Struktur so erfolgt, dass sich die körperkonturkonforme Matrix (40) unmittelbar an die anatomische Struktur anschließt.

4. Optisches Bewegungserfassungssystem (10) nach Anspruch 1, wobei eine Bewegung durch die anatomische Struktur zumindest eine lineare Bewegung der anatomischen Struktur, eine Winkelbewegung durch die anatomische Struktur, eine strukturelle Flexion der anatomischen Struktur, eine strukturelle Extension der anatomischen Struktur, eine strukturelle Expansion der anatomischen Struktur oder eine strukturelle Kompression der anatomischen Struktur beinhaltet.

5. Optisches Bewegungserfassungssystem (10) nach Anspruch 1, wobei die körperkonturkonforme Matrix (30) aus der Gruppe, umfassend einen Tubus, eine Bandage, ein Pessar sowie einen Katheterballon, ausgewählt wird.

6. Optisches Bewegungserfassungssystem (10) nach Anspruch 1, wobei ein innerhalb der körperkonturkonformen Matrix (30) eingebetteter Teil der optischen Faser (40) eine flexible Form aufweist, die auf eine reziproke Bewegung durch die körperkonturkonforme Matrix (30) während der Bildgebung der anatomischen Struktur reagiert.

7. Optisches Bewegungserfassungssystem (10) nach Anspruch 1,
wobei ein innerhalb der körperkonturkonformen Matrix (30) eingebetteter erster Teil der optischen Faser (40) eine inflexible Form aufweist, die nicht auf eine reziproke Bewegung durch die körperkonturkonforme Matrix (30) während der Bildgebung der anatomischen Struktur reagiert, und
wobei ein zweiter Teil der optischen Faser (40) außerhalb der körperkonturkonformen Matrix (30) eine flexible Form aufweist, die auf eine reziproke Bewegung durch die körperkonturkonforme Matrix (30) während der Bildgebung der anatomischen Struktur reagiert.

8. Medizinisches Bildgebungssystem zur Bildgebung einer anatomischen Struktur, wobei das medizinische Bildgebungssystem umfasst.
ein optisches Bewegungserfassungssystem (10) nach Anspruch 1; sowie ein Bildgebungssystem (60),
wobei der Motion-Tracker (50) so eingerichtet ist, dass er Motion-Tracking-Daten (54) erzeugt, die auf eine Bewegung durch die anatomische Struktur während der Bildgebung der anatomischen Struktur schließen lassen, und
wobei das Bildgebungssystem (60) auf die Motion-Tracking-Daten (54) reagiert, um eine Ausführung eines Bewegungsausgleichsalgorithmus durch das Bildgebungssystems (60) zu erleichtern.

9. Medizinisches Bildgebungssystem nach Anspruch 8, wobei eine Bewegung durch die anatomische Struktur zumindest eine lineare Bewegung der anatomischen Struktur, eine Winkelbewegung durch die anatomische Struktur, eine strukturelle Flexion der anatomischen Struktur, eine strukturelle Extension der anatomischen Struktur, eine strukturelle Expansion der anatomischen Struktur oder eine strukturelle Kompression der anatomischen Struktur beinhaltet.

10. Medizinisches Bildgebungssystem nach Anspruch 8, wobei die körperkonturkonforme Matrix (30) aus der Gruppe, umfassend einen Tubus, eine Bandage, ein Pessar sowie einen Katheterballon, ausgewählt wird.

11. Medizinisches Bildgebungssystem nach Anspruch 8, wobei der Motion-Tracker (50) weiterhin so eingerichtet ist, dass er Steuerdaten (55a) des Regelkreises erzeugt, um eine Steuerung des Bildgebungssystems (60) im geschlossenen Kreis zu erleichtern.

12. Medizinisches Bildgebungssystem nach Anspruch 8, das weiterhin umfasst:
ein Rückmeldungssystem (70), wobei der Motion-Tracker (50) weiterhin so eingerichtet ist, dass er Rückmeldungsdaten (55b) erzeugt, um zumindest eine visuelle Rückmeldung sowie akustische Rückmeldung vorzusehen, die auf eine Bewegung der anatomischen Struktur schließen lässt.

13. Medizinisches Bildgebungssystem nach Anspruch 12, wobei zumindest die visuelle Rückmeldung sowie akustische Rückmeldung eine inakzeptable Bewegung durch die anatomische Struktur erkennen lässt.

14. Medizinisches Bildgebungssystem nach Anspruch 8, das weiterhin umfasst:
ein Rückmeldungssystem (70), wobei der Motion-Tracker (50) weiterhin so eingerichtet ist, dass er patientenbezogene Steuerungsdaten (55c) erzeugt, um zumindest die anatomische Struktur zu positionieren oder eine Positionierung der anatomischen Struktur beizubehalten.

15. Medizinisches Bildgebungssystem nach Anspruch 8, wobei der Motion-Tracker (50) weiterhin so eingerichtet ist, dass er Systemsteuerungsdaten (55d) erzeugt, um mindestens einen betrieblichen Aspekt des Bildgebungssystems einzustellen(60).

## Revendications

1. Système optique de détection de mouvement (10) pour l'utilisation dans l'imagerie d'une structure anatomique, le système optique de détection de mouvement (10) comprenant :
un capteur optique de mouvement (20) comprenant une matrice de conformation de contour corporel (30) et une fibre optique (40),
dans lequel, lorsque la matrice de conformation de contour corporel (30) est juxtaposée à la structure anatomique, la matrice de conformation de contour corporel (30) se conforme structurellement au moins partiellement à un contour de surface de la structure anatomique pour réciproquer tout mouvement par la structure anatomique, et
dans lequel la fibre optique (40) est au moins partiellement incorporée dans la matrice de conformation de contour corporel pour générer un signal optique encodé (42) indicatif d'une forme de la fibre optique (40) en réponse à un quelconque mouvement réciproque par la matrice de conformation de contour corporel (30) durant une imagerie de la structure anatomique ; et **caractérisé par**
un organe de suivi de mouvement (50) répondant au signal optique encodé (42) adapté pour reconstruire périodiquement la forme géométrique de la fibre optique (40),
dans lequel chaque changement d'une forme de la fibre optique (40) entre les reconstructions géométriques de la fibre optique (40) représente le mouvement par la structure anatomique durant l'imagerie de la structure anatomique.

2. Système optique de détection de mouvement (10) selon la revendication 1, dans lequel une juxtaposition de la matrice de conformation de contour corporel (30) à la structure anatomique comprend l'apposition de la matrice de conformation de contour corporel (30) à la structure anatomique.

3. Système optique de détection de mouvement (10) selon la revendication 1, dans lequel une juxtaposition de la matrice de conformation de contour corporel (30) à la structure anatomique comprend la contiguïté de la matrice de conformation de contour corporel (30) à la structure anatomique.

4. Système optique de détection de mouvement (10) selon la revendication 1, dans lequel un mouvement par la structure anatomique comprend au moins un parmi un mouvement linéaire de la structure anatomique, un mouvement angulaire par la structure anatomique, une flexion structurelle de la structure anatomique, une extension structurelle de la structure anatomique, un agrandissement structurel de la structure anatomique et une compression structurelle de la structure anatomique.

5. Système optique de détection de mouvement (10) selon la revendication 1, dans lequel la matrice de conformation de contour corporel (30) est sélectionnée parmi un groupe comprenant une gaine, un bandage, un chapeau et un ballonnet de cathéter.

6. Système optique de détection de mouvement (10) selon la revendication 1, dans lequel une partie de la fibre optique (40) incorporée à l'intérieur de la matrice de conformation de contour corporel (30) présente une forme flexible répondant à un quelconque mouvement réciproque par la matrice de conformation de contour corporel (30) durant l'imagerie de la structure anatomique.

7. Système optique de détection de mouvement (10) selon la revendication 1,
dans lequel une première partie de la fibre optique (40) incorporée à l'intérieur de la matrice de conformation de contour corporel (30) présente une forme inflexible ne répondant à aucun mouvement réciproque par la matrice de conformation de contour corporel (30) durant l'imagerie de la structure anatomique, et
dans lequel une seconde partie de la fibre optique (40) externe à la matrice de conformation de contour corporel (30) présente une forme flexible répondant à un quelconque mouvement réciproque par la matrice de conformation de contour corporel (30) durant l'imagerie de la structure anatomique.

8. Système médical d'imagerie pour imager une structure anatomique, le système médical d'imagerie comprenant :
un système de détection de mouvement optique selon la revendication 1 ; et
un système d'imagerie (60),
dans lequel l'organe de suivi de mouvement (50) est utilisable pour générer des données de suivi de mouvement (54) représentatives d'un quelconque mouvement par la structure anatomique durant l'imagerie de la structure anatomique, et
dans lequel le système d'imagerie (60) répond aux données de suivi de mouvement (54) pour faciliter une exécution d'un algorithme de compensation de mouvement par le système d'imagerie (60).

9. Système médical d'imagerie selon la revendication 8, dans lequel un mouvement par la structure anatomique comprend au moins un parmi un mouvement linéaire de la structure anatomique, un mouvement angulaire par la structure anatomique, une flexion structurelle de la structure anatomique, une extension structurelle de la structure anatomique, un agrandissement structurel de la structure anatomique et une compression structurelle de la structure anatomique.

10. Système médical d'imagerie selon la revendication 8, dans lequel la matrice de conformation de contour corporel (30) est sélectionnée parmi un groupe comprenant une gaine, un bandage, un chapeau et un ballonnet de cathéter.

11. Système médical d'imagerie selon la revendication 8, dans lequel l'organe de suivi de mouvement (50) est en outre utilisable pour générer des données de commande à boucle fermée (55a) pour faciliter une commande à boucle fermée du système d'imagerie (60).

12. Système médical d'imagerie selon la revendication 8, comprenant en outre :
un système de rétroaction (70), dans lequel l'organe de suivi de mouvement (50) est en outre utilisable pour générer des données de rétroaction (55b) pour fournir au moins une rétroaction visuelle et une rétroaction audio indicatives d'un mouvement de la structure anatomique.

13. Système médical d'imagerie selon la revendication 12, dans lequel les au moins une rétroaction visuelle et une rétroaction audio indiquent un mouvement inacceptable par la structure anatomique.

14. Système médical d'imagerie selon la revendication 8, comprenant en outre :
un système de rétroaction (70), dans lequel l'organe de suivi de mouvement (50) est en outre utilisable pour générer des données de commande de patient (55c) pour au moins un parmi le positionnement de la structure anatomique et le maintien d'un positionnement de la structure anatomique.

15. Système médical d'imagerie selon la revendication 8, dans lequel l'organe de suivi de mouvement (50) est en outre utilisable pour générer des données de commande de système (55d) pour régler au moins un aspect de fonctionnement du système d'imagerie (60).
